# NEW EUROPEAN PATENT SPECIFICATION

(11) **EP 0 385 296 B2**
(45) Date of publication and mention of the opposition decision: **01.04.1998**
(45) Mention of the grant of the patent: 03.05.1995
(21) Application number: 90103547.7
(22) Date of filing: 23.02.1990
(51) Int. Cl.: A01H 1/02

(54) **Method of producing hybrid allium plant**
Verfahren zur Produktion von Hybridpflanzen von Allium
Méthode de production d'une plante hybride d'allium

(30) Priority: 27.02.1989 JP 47463/89
(43) Date of publication of application: 05.09.1990
(73) Proprietor: PIAS CORPORATION, Osaka (JP)
(72) Inventor: Shimasue, Yoshiyuki, c/o Pias Corporation, Osaka (JP); Ishizuka, Kazuhiro, c/o Pias Corporation, Osaka (JP); Fukuda, Misa, c/o Pias Corporation, Osaka (JP)
(74) Representative: Reinhard - Skuhra - Weise & Partner

(56) References cited:
- EP-A- 0 263 017
- DE-C- 3 248 379
- CAB-Plant Breeding Abstracts Quest nr.80216994 0P050-05693& P.Havranek: "Isolation, cultivation and fusion of protoplasts of common garlik(Alliumsativum) and leek (Allium porrum) Sbornik Mezinarodniho Symposia 6-11 zari 1976,pp.
- Science vol. 219, 11 February 1983, page 683 J.F.Shepard et al: "Genetictransfer in plants though interspecific protoplastfusion"
- CAB-Plant Breeding Abstracts Quest nr.76A31210 0P046-06835& J.C.Bak: "Interspecific fusion of protoplasts between Allium fistulosum and A.cepaKorean Journal of Plant tissue Culture vol.2 nr.1 1974, pp.1-6."
- Physiologia Plantarum vol. 53, 1981, Copenhagen pages 64 - 70; J.Vienken et al:"Electric field induced fusion of isolated vacuoles and protoplasts ofdifferent developmental and metabolic provenience"
- Planta vol. 115, 1974, Berlin&& M.R.Michayluk: "A method for high-frequency intergenericfusion of plant protoplasts"
- Advances in Genetics vol. 20, 1979, pages 151 - 166; I.K.Vasil et al: "Planttissue cultures in genetics and plant breeding; par.IV: Plant protoplasts in genetics and breeding"
- Eucarpia Proceedings 4th Allium Symposium 1988 1988, page 99 E.Baumunk-Wende:"Application of tissue culture in hybridisation of Allium porrum L."
- "Method for production of interspecific hybrids within Brassiceae via somatic hybridization, using resynthesis of Brassica Napus as a model", Plant Science, 43 (1986), pp. 155-162, Elsevier Scientific Publishers Ireland Ltd.
- "The introduction of CMS mitochondria to triazine tolerant Brassica napus L., var. 'Regent", by micromanipulation of individual heterokaryons", Plant Cell Reports (1986)5, pp. 415-418
- "Concanavalin A Improves Polyethylene Glycol Method for Fusing Plant Protoplasts", Physiol. Plant. 44:92-96, 1978
- "Somatic hybrid potato plants after electrofusion of diploid Solanum tuberosum and Solanum phureja", Plant Cell Reports (1986)5:262-265
- "Transfer and segregation of triazine tolerant chloroplasts in Brassica napus L.", Theor Appl Genet(1988) 76:165-171, p. 167
- "Chromosomal Behaviour in Somatic Hybrids of Soybean-Nicotiana glauca", Molec. gen. Genet. 150, 225-230(1977)
- "Soc. Ital. Genetica Agraria - Convegno 1982, pp. 170, 171

## Description

This invention relates generally to a method to produce a hybrid Allium plant for improvement of breed.

Generally, the Allium plants are classified into two different groups which include the so-called seed breeding type and the vegetative propagation type. The Allium plants belonging to the seed breeding type reproduce through sexual crossing, and include onion (Allium cepa) and Welsh onion for example. The vegetatively propagating Allium plants reproduce by the intermediary of bulbs, and include garlic (Allium sativum) and scallion (Allium ascalonicum) for example.

The two types of Allium plants are known to exhibit drastically different characters. Therefore, it is greatly beneficial to introduce the inheritable characters of one type of Allium plants into the other type Allium plants. Further, in view of breeding improvement, such character introduction should be preferably conducted between plants of the same type, either the seed breeding type or the vegetative propagation type, as well as between variant plants of the same species.

A typical method for inter-plant character introduction is the cross-fertilization. However, this known method is disadvantageous in that it is only applicable to closely similar plants in addition to requiring much time and labor for crossing or breeding procedures. More significantly, it is impossible to utilize the cross-fertilization in plants of the vegetative propagation nature.

Opatrny, Z. and Havranek, P. ("Isolation, cultivation and fusion of protoplasts of common garlic (Allium sativum) and leek (Allium porrum) Sbornik Mezinarodniho Symposia 6-11 zari 1976, pp. 471-476") disclose the isolation of protoplasts from leek and garlic followed by fusion of the respective protoplasts. However, the growth of such fused protoplasts to hybrid plants is not described. Furthermore, hybrid protoplasts between garlic and onion are not mentioned.

The method of regenerating intact plants from protoplast fusions is only established for dicotyledonic plants, but not for monocotyledons like Allium species ( Römpp Lexikon Biotechnologie, Georg Thieme Verlag Stuttgart, New York, 1992, pages 633 and 634, Keyword "Protoplasten"). Such regeneration of fused hybrid protoplasts of monocotyledons to complete hybrid plants was successful only with gramineous hybrid plants of rice, but not with Allium species (EP 0 263 017).

It is, therefore, an object of the present invention to provide a hybridizing technique for the Allium plants onion and garlic which can be applied to various combinations of such Allium plants for character introduction or mixing particularly with respect to Allium plants of the vegetative propagation nature.

To fulfill these objects, the present invention provides a method of producing a hybrid Allium plant according to claim 1.

Obviously, the use of cell fusion makes the method of the present invention applicable to a combination of the Allium plants onion and garlic. Particularly, the method is advantageous in that it may be used even when one of the starting plants is of the vegetative propagation nature, as opposed to the conventional cross-fertilization method which is limited in applicability to plants of the seed breeding nature. Further, the method may be applied to a combination of different variants of onion and garlic species.

Another advantage of the present invention is that the method is free of seasonal limitations, and requires less time and labor than the conventional cross-fertilization method.

As a result of the present invention, it is possible to produce a hybrid Allium plant which has the respective characters of the two different starting plants onion and garlic. More specifically, the following results may be obtained.
(1) In the combination of onion and garlic:
   (a) It is possible to introduce the effective ingredients (allicin for example) of the garlic into the onion.
   (b) The garlic can be made to have the cold resistance and hot weather resistance of the onion.
   (c) The garlic, which is of the vegetative propagation nature, can be made to have the sexual reproducibility which is provided by the onion.

The present invention will now be described further, by way of example only, with reference to the accompanying drawings, in which:
Figure 1 is a plan view showing a gelled agarose-based culture medium which has been cut;
Figure 2a is a plan view in section of a nursing culture system;
Figure 2b is a front view in section of the nursing culture system shown in Figure 2a; and
Figure 3 is a plan view of a callus forming culture medium.

As described hereinbefore, the method according to the present invention is applicable to a combination of the Allium plants onion and garlic. Examples of the Allium plants include onion (Allium cepa), garlic (Allium sativum).

The method of the present invention comprise four main steps. Two different protoplast donors are prepared from the two different Allium plants onion and garlic in a first step which is followed by a second step wherein protoplasts are prepared from the protoplast donors. In a third step, the prepared protoplasts are subjected to cell fusion to provide hybrid cells. The hybrid cells are cultured to complete plant in a fourth step which may include incubation of the hybrid cells for callus formation prior to growth to complete plant.

Regarding the first step, each protoplast donor may be taken from various portions of these starting Allium plants, grown or non-grown. Examples of the protoplast donor include a normal grown plant, a sterilized young plant obtained by incubating a leaf generating portion of a bulb, a plant derived from a growth point, a plant obtained by redifferentiation of callus, callus derived from a growth point, embryo-derived callus, and callus derived from other organs or tissues of a plant.

Particularly, when a growth-point-derived plant, growth-point-derived callus or embryo-derived callus is used as a protoplast donor, the hybrid cells obtained therefrom show a high potential of growth and/or differentiation. Further, when a growth-point-derived plant or growth-point-derived callus is used as a protoplast donor, the resulting hybrid plant can be made virus-free.

In the second step, both protoplast donors are treated by an enzyme solution to prepare respective protoplasts. The composition of the enzyme solution may be optionally determined depending on the kind of the protoplast donor or the starting plant.

In the third step, the respective protoplasts are subjected to cell fusion by using a known cell fusion technique. As a result, hybrid cells are formed in a background of non-fused cells and fused non-hybrid cells.

After the cell fusion, the hybrid cells are sorted out in accordance with claim 1 and cultured separately from the other cells. Such cell sorting may be conducted visually in the visible region of light by the use of a microscope. The cell sorting may also be performed by the fluorescence microscopy wherein a fluorescence microscope enables distinction of the fused cells by coloring thereof under irradiation of blue excitation light.

The visible region cell sorting is described more in detail. It is now assumed that one of the protoplast donors is callus, whereas the other protoplast donor is a leaf blade of a plant. In such a combination, the protoplasts resulting from the callus are featured by being cytoplasm-rich (large cytoplasm with small vacuoles), while the protoplasts obtained from the leaf blade are characterized by the presence of chloroplasts which are green in the visible region of light. Thus, the wanted hybrid cells can be visually distinguished from the non-fused cells and the fused non-hybrid cells by the dual features of being rich in cytoplasm and having green chloroplasts.

However, it is possible that the resulting hybrid cells do not contain an enough number of chloroplasts, thereby failing to provide a distinct green color feature. In this case, the hybrid cells cannot be readily distinguished from the non-fused cytoplasm-rich cells or the fused non-hybrid cytoplasm-rich cells.

According to the fluorescence microscopy cell sorting method described above, the cell mixture is subjected to irradiation of blue excitation light to cause red coloring of the chloroplasts. Thus, under this condition, the hybrid cells can be readily distinguished from the other cytoplasm-rich cells by such coloring of the former.

It should be appreciated that the cytoplasm-rich hybrid cells can be readily distinguished from the non-fused leaf blade cells or fused non-hybrid lead blade cells which are not cytoplasm-rich. Alternatively, the hybrid cells may be later sorted from the non-hybrid leaf blade cells by selecting a suitable culture medium.

Prior to conducting the cell sorting, the hybrid protoplasts together with the non-fused protoplasts should be cultured to such an extent that cell walls are regenerated. This is because the regenerated cell walls protect against cell damaging upon picking up the subsequently sorted hybrid cells.

Further, after the cell sorting and picking up, the sorted hybrid cells may be preferably transferred into a nursing culture system which is used for incubating the hybrid cells prior to culturing thereof to complete plant. Specifically, the nursing culture system comprises a filter ring arranged within a Petri dish which contains, around the filter ring, protoplasts of an Allium plant having a high growing ability to serve as a nursing agent. The nursing protoplasts assist cell division of the hybrid cells.

In an alternative embodiment, the hybrid cells obtained as a result of the preceding cell fusion may be incubated for callus formation together with non-hybrid cells (non-fused cells and fused non-hybrid cells), and thereafter sorted out from the non-hybrid cells for growth to complete plant. In other words, the hybrid cells may be separated from the non-hybrid cells at the callus stage by utilizing different features of differently originated calluses. For example, the callus originated from garlic cells is known to be relatively compact (containing finer cells in a dense state) due to its high redifferentiation capability. The garlic callus is also featured by its yellow color. On the other hand, the onion callus tends to be friable (containing coarser cells) due to redifferentiation inability, and lacks a yellow color feature.

More specifically, it is now assumed that, in incubating onion-garlic hybrid cells together with onion cells (non-fused onion cells and fused non-hybrid onion cells) and garlic cells (non-fused garlic cells and fused non-hybrid garlic cells), a culture medium is selected which allows growth of the onion cells and the hybrid cells (because of the inherited onion features) while prohibiting growth of the garlic cells. In this case, only two kinds of call (or calluses) are formed which include onion-cell-derived callus and hybrid-cell-derived callus. Of these, the hybrid-cell-derived callus is characterized in that it is compact and yellow because of the inherited garlic features. Thus, the hybrid callus can be easily sorted out from the onion callus before subsequent growth to complete plant.

The present invention will now be described further on the basis of more specific examples.

### EXAMPLE I

This example refers to a combination of onion and garlic. The process was conducted in the following order.

### (I-1) Preparation of Protoplast Donors:

Protoplast donors were separately prepared with respect to onion and garlic.

First, an onion seed and a garlic bulb were sterilized by substantially the same method which includes the following sub-steps.
(I-1-a) Using a brush, the onion seed and the garlic bulb were separately washed with a neutral detergent. The onion seed alone was wrapped in gauze.
(I-1-b) The seed and the bulb were immersed in sterile water for 15-20 minutes for defoaming, and thereafter washed supersonically in 2% benzalkonium chloride solution (available from Daigo Eiyo Chemical Co., Inc., Japan) for 15 minutes.
(I-1-c) The seed and the bulb were washed three times in sterile water, and thereafter immersed in 70% ethanol for 30-60 seconds. Subsequently, they were again washed three times in sterile water.
(I-1-d) The seed and the bulb were supersonically washed for 15 minutes in 5% aqueous solution containing 2% sodium hypochloride solution (available from Kanto Chemical Co., Inc., Japan), such supersonic washing being conducted twice.
(I-1-e) Finally, the seed and the bulb were washed at least six times in sterile water.

For preparing the onion protoplast donor, the sterilized onion seed was cultured in a modified BDS medium [1% agar medium containing 2,4-dichlorophenoxyacetic acid (hereafter abbreviated to 2,4-D) with molarity of 1.25x10⁻⁵ M] having the composition shown in the following Table 1 (see the next page). As a result, callus was derived from the growth point of a leaf generating portion of the seed. The thus derived callus was subcultured in a modified BDS medium (liquid medium containing 2.5x10⁻⁶ M 2,4-D). Two-day-old subcultured callus was used as the onion protoplast donor.

It should be understood that all the culture media used in the examples of the present invention were adjusted to pH5.6 with NaOH and subjected to filter sterilization.

**TABLE 1**

| Composition of Modified BDS Culture Medium | | |
|---|---|---|
| Compound | Weight/l | Concentration |
| CaCl₂.2H₂O | 150mg | 1.02mM |
| KNO₃ | 2.53mg | 25.02mM |
| NH₄NO₃ | 320.16mg | 4.0mM |
| NH₄H₂PO₄ | 230.06mg | 2.0mM |
| (NH₄)₂SO₄ | 134mg | 1.01mM |
| MgSO₄.7H₂O | 247mg | 1.00mM |
| MnSO₄.4H₂O | 13.2mg | 0.045mM |
| ZnSO₄.7H₂O | 2.0mg | 6.95 µM |
| CuSO₄.5H₂O | 0.039mg | 0.1 µM |
| KI | 0.75mg | 4.52 µM |
| CoCl₂.6H₂O | 0.025mg | 0.105 µM |
| H₃BO₃ | 3.0mg | 0.049mM |
| Na₂MoO₄.2H₂O | 0.25mg | 1.03 µM |
| NaH₂PO₄.2H₂O | 172mg | 1.04 µM |
| FeSO₄.7H₂O | 27.85mg | 100 µM |
| Na₂EDTA | 37.25mg | 100 µM |
| Nicotinic acid | 1.0mg | 8.1 µM |
| Thiamine HCl | 10mg | 0.03mM |
| Pyridoxine HCl | 1.0mg | 4.9 µM |
| meso-Inositol | 100mg | 1.8mM |
| Sucrose | 30g | 0.088M |
| Casamino acid | | 0.03% |

For preparing the garlic protoplast donor, a leaf generating portion was excised with a scalpel from the sterilized garlic bulb, and thereafter cultured for about 1-2 weeks in a modified BDS medium [hormone-free 0.2% Gellan Gum medium (available from Kelco Division of Merck & Co., Inc., Japan)]. A sterile young plant resulted from such culturing. A leaf blade of the young plant was used as the garlic protoplast donor.

### (I-2) Preparation of Protoplasts:

In the second process step, refined protoplasts were prepared respectively from the onion protoplast donor (cultured callus suspension) and the garlic protoplast donor (young plant leaf blade).

### (I-2-A) Preparation of Onion Protoplasts:

The onion protoplasts were prepared in the following way.
(I-2-A-a) The supernant of the onion culture suspension was removed, and about 0.5g of the onion callus (cell masses) was suspended in an enzyme solution (about 25ml) having the composition shown in the following Table 2. In Table 2, "MES" is an abbreviation of 2-(N-morpholino) ethane-sulfonic acid, whereas the term "MES Good's buffer" means a buffer solution obtained by dissolving 4.265g of 20mM MES.H₂O and 1.017g of 5mM MgCl₂.6H₂O in distilled water (total volume being 1 liter) and thereafter adjusting the solution to pH5.6 with NaOH.

**TABLE 2**

| Composition of Enzyme Solution for Onion Protoplast Donor | |
|---|---|
| Cellulase "Onozuka" R-10 (Yakult Honsha Co., Ltd., Japan) | 1.0% |
| Macerozyme R-10 (Yakult Honsha Co., Ltd., Japan) | 0.5% |
| Driselase (Kyowa Hakko Kogyo Co., Ltd., Japan) | 0.5% |
| Mannitol | 0.6M |
| MES Good's buffer (pH5.6) | |

(I-2-A-b) The cell liquid obtained above was shaked for about 5-6 hours by a gyratory shaker (100rpm).
(I-2-A-c) The cell liquid was passed moderately through sieves of progressively decreasing mesh sizes (mesh sizes decreasing in the order of 150 µm, 90 µm, 60 µm, and 40 µm). As a result, coarser cells were removed, while wanted finer cells remained with the liquid.
(I-2-A-d) The filtered cell liquid was transferred into a precipitation tube after passing through another 40 µm sieve, and subjected to centrifugation for 3 minutes at 600rpm.
(I-2-A-e) The supernant of the centrifuged cell liquid was removed, and the sedimented cells were suspended in a washing liquid. This washing liquid was prepared by dissolving 22.77g of 0.5M mannitol and 0.09g of 2.5mM CaCl₂ in distilled water (total volume being 250ml) and thereafter adjusting the solution to pH5.6.
(I-2-A-f) The liquid obtained by the sub-step (I-2-A-e) was placed in a 10ml precipitation tube and subjected to centrifugal separation for 3 minutes at 600rpm, the centrifugal separation being conducted three times.
(I-2-A-g) Finally, the supernant of the centrifuged cell liquid was removed to provide refined onion protoplasts.

### (I-2-B) Preparation of Garlic Protoplasts:

The garlic protoplasts were prepared, as follows.
(I-2-B-a) The leaf blade of the sterile garlic young plant was cut with a scalpel into square fragments (about 3mm in size).
(I-2-B-b) The obtained fragments were suspended in an enzyme solution (about 25ml) having the composition shown in the following Table 3.

**TABLE 3**

| Composition of Enzyme Solution for Garlic Protoplast Donor | |
|---|---|
| Cellulase "Onozuka" R-10 (Yakult Honsha Co., Ltd., Japan) | 2.0% |
| Macerozyme R-10 (Yakult Honsha Co., Ltd., Japan) | 0.5% |
| Driselase (Kyowa Hakko Kyogyo Co., Ltd., Japan) | 1.0% |
| Mannitol | 0.6M |
| MES Good's buffer (pH5.6) | |

(I-2-B-c) The suspension obtained above is shaked for about 5-6 hours by a gyratory shaker (50rpm).

Subsequently, sub-steps similar to the sub-steps (I-2-A-c) to (I-2-A-g) for the onion protoplasts were performed to provide refined garlic protoplasts.

### (I-3) Cell Fusion:

In the third step, the two kinds of protoplasts were subjected to cell fusion. For this purpose, two kinds of cell suspensions were prepared which respectively contained onion protoplasts and green garlic protoplasts at a concentration of 2x10⁵ cells/ml. The cell suspensions were then mixed together at a ratio of 1:1, and 0.8ml of the mixture was subjected to cell fusion by using Shimazu Cell Fusion Apparatus SSH-1 (Shimazu Corp., Japan). The cell fusion was conducted under the conditions shown in the following Table 4.

**TABLE 4**

| Conditions for Cell Fusion | |
|---|---|
| Frequency of alternating current | 1MHz |
| Voltage of initial alternating current (VAC) | 40V |
| Intial VAC time | 10sec |
| Pulse width for cell fusion | 30microsec |
| Voltage of cell fusion pulse (VDC) | 200V |
| Field strength | 1.00KV/cm |
| Pulse interval | 3sec |
| Pulse number | 3 |
| VDC decrease rate | 80% |
| Voltage of secondary alternating current applied during pulse interval | 35V |
| Time for final alternating current | 15sec |
| Voltage decrease rate of final alternating current | 90% |

### (I-4) Incubation:

The protoplast suspension obtained by the preceding process step was subjected to centrifugal separation, and the supernant was removed. The resulting precipitate, which consisted of fused cells (hybrid cells and fused non-hybrid cells) and non-fused cells, was suspended in a modified 8p culture medium (containing 2.5x10⁻⁶ M 2,4-D) having the composition shown in the following Table 5 (see next page). 0.5ml of the thus obtained suspension was mixed with 0.5ml of another modified 8p culture medium [containing 2.5x10⁻⁶ M 2,4-D and 0.6% Sea Plaque agarose (FMC Corporation)] having been maintained at a temperature of 40°C. The mixture was then placed into a Petri dish of 35mm diameter and allowed to solidity, the mixture having a protoplast density of about 1x10⁵ cells/ml. Thereafter, the solidified mixture was incubated stationarily at 25°C in the dark for a period of about 10 days. During this stationary incubation, the modified 8p medium was supplemented every 4-5 days with about 0.05ml of a modified BDS medium having the composition already shown in Table 1.

It should be appreciated that the modified 8p medium was selected for the incubation because it prohibits colony formation with respect to the non-fused garlic protoplasts and the fused non-hybrid garlic protoplasts while allowing cell division of the onion protoplasts (the non-fused onion protoplasts and the fused non-hybrid onion protoplasts) and the hybrid protoplasts to enable colony formation.

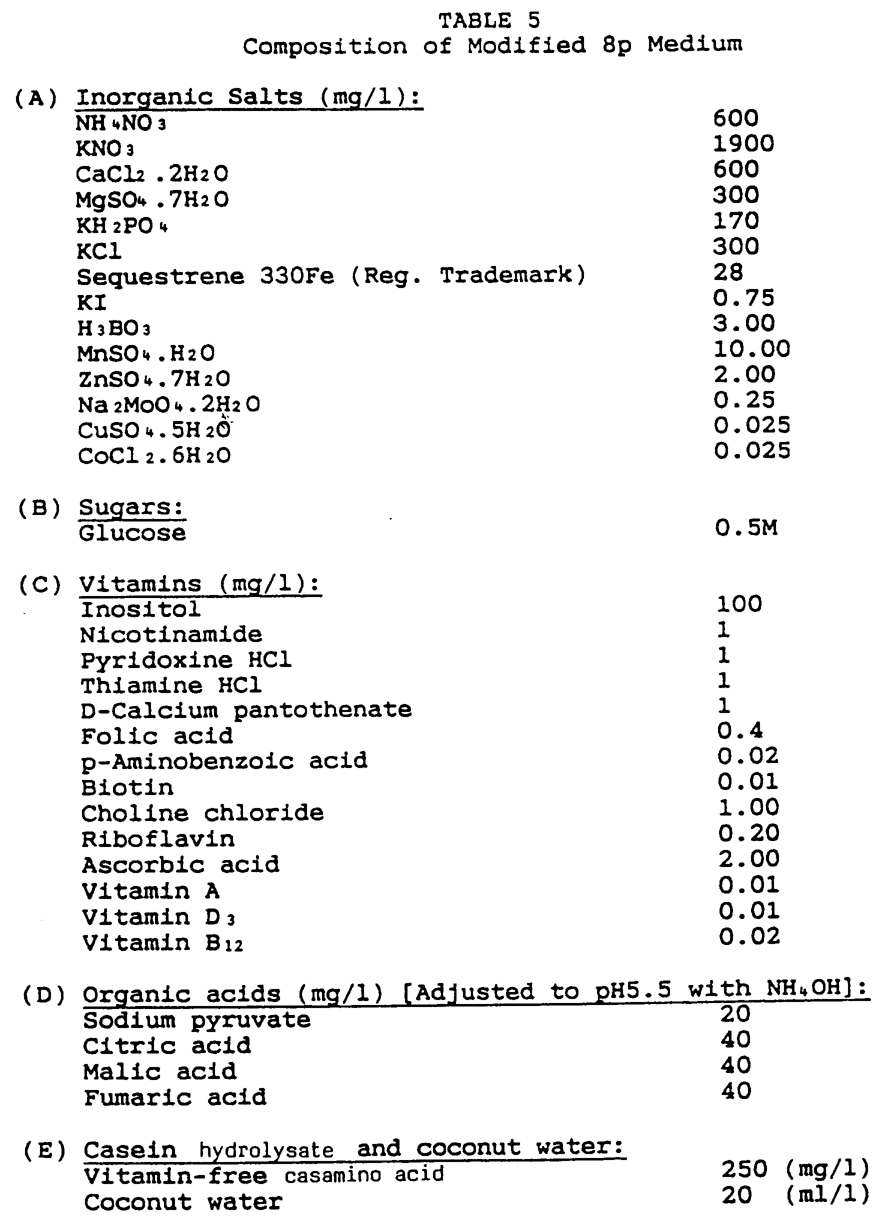

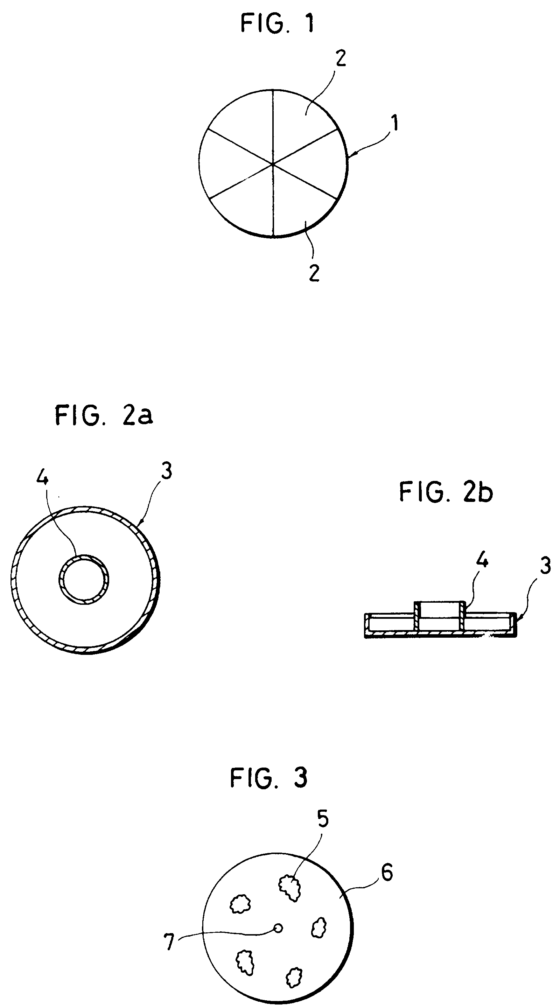

After the stationary incubation, the agarose gel 1 was cut into pieces 2, as shown in Figure 1. Three of the cut pieces 2 were placed in a Petri dish (35mm diameter) containing 0.8ml of a modified BDS liquid medium (see Table 1). Thereafter, the culture system was subjected to shaking (50rpm) at 25°C in the dark for a period of about 30 days.

After colonial formation, the formed colonies were transferred into a modified BDS medium (containing 2.5x10⁻⁶ M 2,4-D and 0.2% Gellan Gum) for stationary culturing at 25°C, thereby forming calli. Of the calli thus formed, compact ones resulted from the hybrid cells, whereas friable ones originated from the onion cells.

### (I-5) Culturing to Complete Plant:

### (I-5-a) Breeding:

One of the compact calli obtained in the foregoing step was placed in a 50ml Erlenmeyer flask which contained 5-10ml of a modified BDS liquid medium, and thereafter shaked at 50rpm. A similar operation was repeated until the callus was sufficiently multiplied.

### (I-5-b) Redifferentiation:

The multiplied callus obtained in the preceding sub-step (I-5-a) was cultured at 25°C under 1,500-2,500 LUX light irradiation in a modified MS medium [containing 0.2% Gellan Gum, 5.4x10⁻⁶ M naphthalenacetic acid (NAA), and 8.9x10⁻⁶ M benzyladenine (BA)] having the composition shown in the following Table 6. As a result, an adventitous bud was formed upon lapse of about 4 months.

**TABLE 6**

| Composition of Modified MS Medium | |
|---|---|
| NH₄NO₃ | 1650mg/l |
| KNO₃ | 1900mg/l |
| CaCl₂.2H₂O | 440mg/l |
| MgSO₄.7H₂O | 370mg/l |
| KH₂PO₄ | 170mg/l |
| KI | 0.83mg/l |
| H₃BO₃ | 6.2mg/l |
| MnSO₄.4H₂O | 22.3mg/l |
| ZnSO₄.4H₂O | 8.6mg/l |
| Na₂MoO₄.2H₂O | 0.25mg/l |
| CuSO₄.5H₂O | 0.025mg/l |
| CoCl₂.6H₂O | 0.025mg/l |
| Na₂EDTA | 37.3mg/l |
| FeSO₄.7H₂O | 27.8mg/l |
| Myo-Inositol | 100mg/l |
| Nicotinic acid | 0.5mg/l |
| Thiamine HCl | 0.1mg/l |
| Pyridoxine HCl | 0.5mg/l |
| Glycine | 2mg/l |
| Sucrose | 3% |
| Casamino acid | 0.03% |

It should be appreciated that the modified MS medium does not allow the onion calli (the non-fused onion cells and the fused non-hybrid onion cells) to generate an adventive bud. Therefore, all of the call (including the onion calli and the hybrid calli) may be cultured together in the modified MS medium wherein the onion calli drop out.

### (I-5-c) Growth to Complete Plant:

In the final stage, the adventive bud obtained as a result of the redifferentiation was transferred into another modified 1/2 MS medium (modified MS medium diluted to a 1/2 concentration) containing 0.2% Gellan Gum for root initiation and generation of a young plant. The young plant thus obtained was then implanted on vermiculite for acclimation, and thereafter transferred to culture earth. As a result, a complete onion-garlic hybrid plant was produced.

### EXAMPLE II

This example differs from Example I only in the following respects.

### (II-i) Difference in Protoplast Preparation:

In this example, the garlic protoplasts were prepared in the following way which is a substitute for the process step (I-2-B) of Example I.
(II-i-a) First, the leaf blade of the sterile garlic young plant was cut with a scalpel into square fragments (about 3mm in size). This sub-step is identical to the sub-step (I-2-B-a) of Example I.
(II-i-b) The obtained fragments were immersed in a modified MS liquid medium (containing 5.4x10⁻⁶ M NAA and 8.9x10⁻⁶ M BA) having the composition shown in Table 6 above.
(II-i-c) The suspension obtained above is shaked for 24 hours at 25°C in the dark (50rpm).

Subsequently, the fragments were transferred into an enzyme solution (see Table 3) and processed in the same manner as in Example I, thereby providing refined garlic protoplasts.

### (II-ii) Difference in Incubation:

The incubating step in this example was conducted in the following manner, as opposed to the process step (I-4) of Example I.
(II-ii-a) The protoplast suspension obtained by the preceding cell fusion [see the process step (I-3) of Example I] was subjected to centrifugal separation, and the supernant was removed. The resulting precipitate, which consisted of fused cells and non-fused cells, was suspended in a modified 8p culture medium (containing 2.5x10⁻⁶ M 2,4-D) having the composition shown in Table 5 above. The thus obtained suspension was transferred into a 35mm diameter Petri dish which had been previously supplied with another modified 8p culture medium (containing 2.5x10⁻⁶ M 2,4-D and 0.2% Gellan Gum). The obtained culture system was then placed in the dark at 25°C, and maintained stationary. As a result, the protoplasts were allowed to have regenerated cell walls.
(II-ii-b) Within 5 days after the cell fusion, those cells (hybrid cells) having the following features were picked up from the culture system of the preceding sub-step (II-ii-a) by using a micropipette having a diameter of 100-150 µm.
   * Cytoplasm-rich and having chloroplasts which are green in the visible region of light.
   * Cytoplasm-rich and having chloroplasts which are colored red under irradiation of blue excitation light.
(II-ii-c) The hybrid cells thus picked up were placed in a membrane filter ring 4 which in turn was arranged centrally in a nursing culture Petri dish 3, as shown in Figures 2a and 2b. The dish 3 was previously supplied with 3ml of a modified 8p medium (containing 2.5x10⁻⁶ M 2,4-D and 1% Sea Plaque agarose), and the membrane filter ring 4 was arranged in place before solidification of the medium.
(II-ii-d) A modified 8p medium (containing 2.5x10⁻⁶ M 2,4-D and 0.3% Sea Plaque agarose) having been maintained at 40°C was dripped into the filter ring 4, and the ring was closed by a cover (not shown).
(II-ii-e) After solidification of the culture medium, 2-2.5ml of an onion protoplast suspension with a cell population of 1x10⁵ cells/ml was spread on the solidified medium outside the membrane filter ring 4. The onion protoplast suspension contained 0.3% Sea Plaque agarose and a modified 8p medium (containing 2.5x10⁻⁶ M 2,4-D).
(II-ii-f) The nursing culture system was then placed in the dark at 25°C for about 10 days, and maintained stationary. During this stationary culturing, 0.15ml of a modified BDS medium (containing 2.5x10⁻⁶ M 2,4-D) was added every 4-5 days to the Petri dish 3 outside the filter ring 4..
(II-ii-g) After the stationary culturing above, the culture system was subjected to shaking at 50rpm for 30 days. This resulted in colony formation.
(II-ii-h) The colony thus formed was transferred to a callus forming culture system comprising another protective culture Petri dish 6, as shown in Figure 3. The Petri dish 6 contained onion call 5 of a high cell division potential which had been previously cultured for 3 days in an agar medium to work as an nursing agent for the hybrid-cell-derived colony 7. The callus forming culture system was maintained stationary at 25°C in the dark for about 14 days, which resulted in formation of a small callus.

Example II is otherwise the same as Example 1.

## Claims

1. A method of producing a hybrid Allium plant comprising the steps of: preparing two different protoplast donors from two different Allium plants, isolating protoplasts from the donors, subjecting the protoplasts to cell fusion to provide hybrid cells, and thereafter culturing the hybrid cells, wherein, after the cell fusion, the hybrid cells are sorted out from the other cells and thereafter cultured separately therefrom, characterized in that:
after the cell fusion and before the cell sorting, the hybrid cells together with the other cells are cultured at least to such an extent that cell walls are regenerated; and the two Allium plants are onion and garlic, respectively.

2. A method as defined in claim 1, wherein, as a preliminary step before the isolation of the protoplasts, each of the protoplast donors is immersed in a culture medium.

3. A method as defined in claim 2, wherein the culture medium for the preliminary step contains at least one of naphthaleneacetic acid (NAA) and benzyladenine (BA).

4. A method as defined in one or more of claims 1 - 3, wherein each of the protoplast donors is selected from the group consisting of a normal grown plant, a sterilized young plant obtained by incubating a leaf generating portion of a bulb, a plant derived from a plant growth point, a plant obtained by redifferentiation of callus, callus derived from a plant growth point, embryo-derived callus, and callus derived from other organs or tissues of a plant other than a growth point and embryo.

5. A method as defined in one or more of claims 1 - 4, wherein the sorting of the hybrid cells is conducted by using a fluorescence microscope which enables distinction of the hybrid cells by coloring thereof under irradiation of blue excitation light.

6. A method as defined in one or more of claims 1 to 5, wherein, after the cell sorting, the hybrid cells are placed into a nursing culture system, the nursing culture system comprising a filter ring arranged within a Petri dish which contains, around the filter ring, nursing protoplasts taken from an Allium plant having a high potential of cell division, the hybrid cells being placed within the filter ring for nurse-assisted cell division.

7. A method as defined in one or more of claims 1 to 6, wherein an initial stage of the hybrid cell culturing is conducted by using a modified 8p culture medium [containing 2,4-dichlorophenoxyacetic acid (2.5x10⁻⁶ M)] having the composition shown in the following Table I, the modified 8p culture medium being progressively supplemented every 4-5 days with a modified BDS culture medium [containing 2,4-dichlorophenoxyacetic acid (2.5x10⁻⁶ M)] having the composition shown in the following Table II. 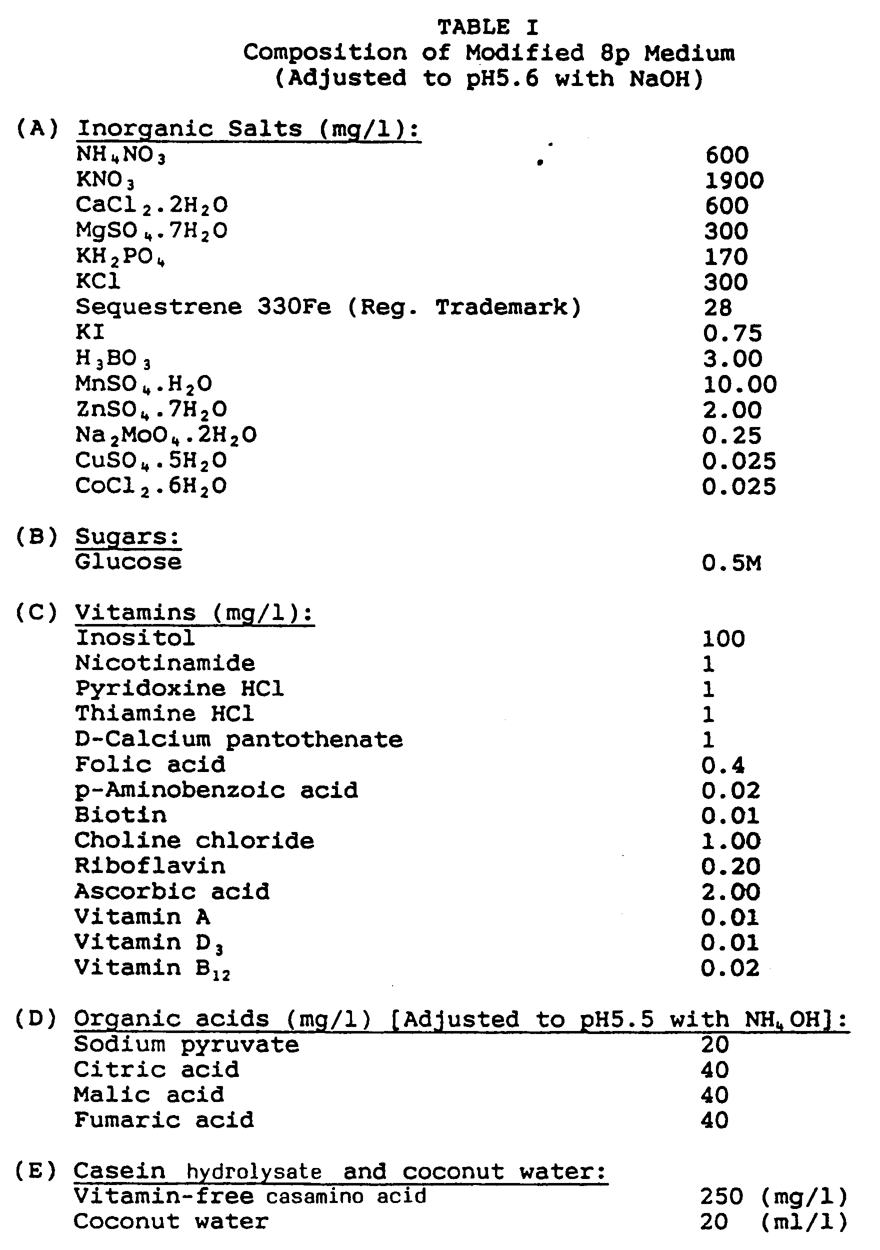
**TABLE II**
| Composition of Modified BDS Culture Medium (Adjusted to pH5.6 with NaOH) | | |
|---|---|---|
| Compound | Weight/l | Concentration |
| CaCl₂.2H₂O | 150mg | 1.02mM |
| KNO₃ | 2.53mg | 25.02mM |
| NH₄NO₃ | 320.16mg | 4.0mM |
| NH₄H₂PO₄ | 230.06mg | 2.0mM |
| (NH₄)₂SO₄ | 134mg | 1.01mM |
| MgSO₄.7H₂O | 247mg | 1.00mM |
| MnSO₄.4H₂O | 13.2mg | 0.045mM |
| ZnSO₄.7H₂O | 2.0mg | 6.95 µM |
| CuSO₄.5H₂O | 0.039mg | 0.1 µM |
| KI | 0.75mg | 4.52 µM |
| CoCl₂.6H₂O | 0.025mg | 0.105 µM |
| H₃BO₃ | 3.0mg | 0.049mM |
| Na₂MoO₄.2H₂O | 0.25mg | 1.03 µM |
| NaH₂PO₄.2H₂O | 172mg | 1.04 µM |
| FeSO₄.7H₂O | 27.85mg | 100 µM |
| Na₂EDTA | 37.25mg | 100 µM |
| Nicotinic acid | 1.0mg | 8.1 µM |
| Thiamine HCl | 10mg | 0.03mM |
| Pyridoxine HCl | 1.0mg | 4.9 µM |
| meso-Inositol | 100mg | 1.8mM |
| Sucrose | 30g | 0.088M |
| Casamino acid | | 0.03% |

## Patentansprüche

1. Verfahren zur Herstellung einer Allium-Hybridpflanze mit den Schritten: Herstelllen von zwei verschiedenen Protoplastendonoren aus zwei verschiedenen Allium-Pflanzen, Isolieren der Protoplasten aus den Donoren, Zellfusion der Protoplasten, um Hybridzellen herzustellen, und anschließend Kultivieren der Hybridzellen, worin, nach der Zellfusion, die Hybridzellen von den anderen Zellen aussortiert werden, und anschließend getrennt hiervon kultiviert werden, **dadurch gekennzeichnet**, daß nach der Zellfusion und vor der Zellsortierung die Hybridzellen zusammen mit den anderen Zellen wenigstens solange kultiviert werden, bis die Zellwände regeneriert sind und wobei die zwei Allium-Pflanzen Zwiebel bzw. Knoblauch sind.

2. Verfahren nach Anspruch 1, worin, als ein vorläufiger Schritt vor der Isolation der Protoplasten, jeder der Protoplastendonoren in ein Kulturmedium eingetaucht wird.

3. Verfahren nach Anspruch 2, worin das Kulturmedium für den vorläufigen Schritt zumindest eine der Verbindungen Naphthalinessigsäure und Benzyladenin enthält.

4. Verfahren nach einem oder mehreren der Ansprüche 1-3, worin jeder der Protoplastendonoren ausgewählt wird aus der Gruppe, bestehend aus einer normal gewachsenen Pflanze, einer sterilisierten jungen Pflanze, die durch Inkubieren eines ein Blatt erzeugenden Teils einer Zwiebel erhalten wurde, einer Pflanze, die aus einem Pflanzenwachstumspunkt stammt, einer Pflanze, die durch Redifferenzierung von Kallus erhalten wurde, Kallus, der aus einem Pflanzenwachstumspunkt stammt, aus Embryonen stammender Kallus, und Kallus, der aus anderen Organen oder Geweben einer Pflanze als einem Wachstumspunkt und einem Embryo stammt.

5. Verfahren nach einem oder mehreren der Ansprüche 1-4, worin die Sortierung der Hybridzellen unter Verwendung eines Fluoreszenzmikroskops durchgeführt wird, das die Unterscheidung der Hybridzellen durch ihre Anfärbung unter Bestrahlung mit einem blau-anregenden Licht ermöglicht.

6. Verfahren nach einem oder mehreren der Ansprüche 1-5, worin, nach der Zellsortierung, die Hybridzellen in ein Ammen-Kultursystem gegeben werden, das Ammen-Kultursystem einen in einer Petrischale angeordneten Filterring aufweist, die, um den Filterring, Nährprotoplasten aus einer Allium-Pflanze mit einem hohen Zellteilungspotential enthält und die Hybridzellen zur von den Ammenzellen unterstützen Zellteilung in den Filterring gegeben werden.

7. Verfahren nach einem oder mehreren der Ansprüche 1-6, wobei auf einer Anfangsstufe der Hybridzelle die Kultivierung unter Verwendung eines modifizierten 8p-Kulturmediums [enthaltend 2,4-Dichlorphenoxyessigsäure (2,5x10⁻⁶ M] mit der in der nachfolgenden Tabelle I gezeigten Zusammensetzung durchgeführt wird, das modifizierte 8p-Kulturmedium fortlaufend alle 4-5 Tage mit einem modifizierten BDS-Kulturmedium [enthaltend 2,4-DichlorphenoxyessigsΣure (2,5x10⁻⁶ M)] mit der in der nachfolgenden Tabelle II gezeigten Zusammensetzung ergänzt wird. 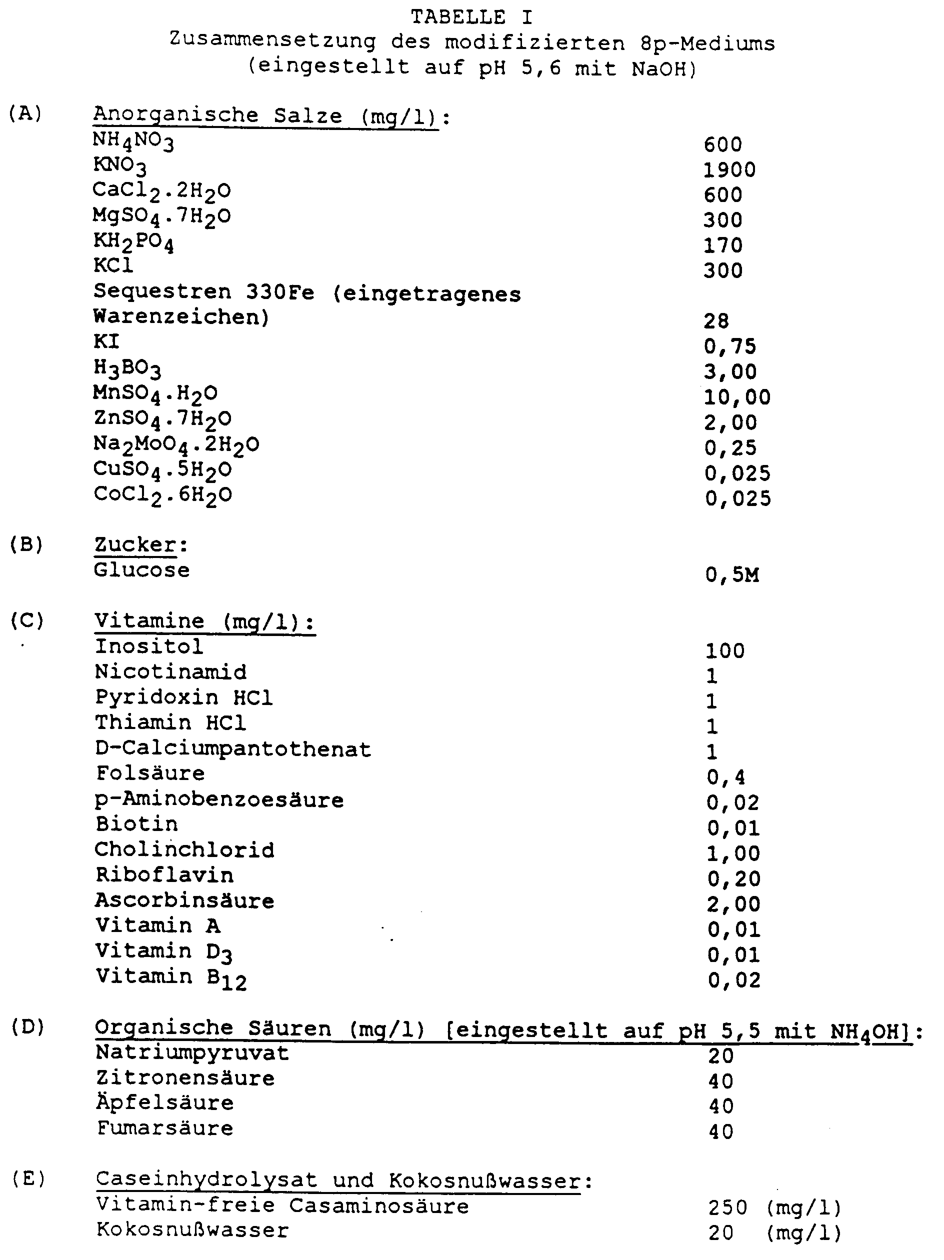
**TABELLE II**
| Zusammensetzung des modifizierten BDS-Kulturmediums (eingestellt auf pH 5,6 mit NaOH) | | |
|---|---|---|
| Verbindung | Gewicht/l | Konzentration |
| CaCl₂.2H₂O | 150 mg | 1,02 mM |
| KNO₃ | 2,53 mg | 25,02 mM |
| NH₄NO₃ | 320,16 mg | 4,0 mM |
| NH₄H₂PO₄ | 230,06 mg | 2,0 mM |
| (NH₄)₂SO₄ | 134 mg | 1,01 mM |
| MgSO₄.7H₂O | 247 mg | 1,00 mM |
| MnSO₄.4H₂O | 13,2 mg | 0,045 mM |
| ZnSO₄.7H₂O | 2,0 mg | 6,95 µM |
| CuSO₄.5H₂O | 0,039 mg | 0,1 µM |
| KI | 0,75 mg | 4,52 µM |
| CoCl₂.6H₂O | 0,025 mg | 0,105 µM |
| H₃BO₃ | 3,0 mg | 0,049 mM |
| Na₂MoO₄.2H₂O | 0,25 mg | 1,03 µM |
| NaH₂PO₄.2H₂O | 172 mg | 1,04 µM |
| FeSO₄.7H₂O | 27,85 mg | 100 µM |
| Na₂EDTA | 37,25 mg | 100 µM |
| Nicotinsäure | 1,0 mg | 8,1 µM |
| Thiamin HCl | 10 mg | 0,03 mM |
| Pyridoxin HCl | 1,0 mg | 4,9 µM |
| Meso-Inositol | 100 mg | 1,8 mM |
| Sucrose | 30 g | 0,088 M |
| Casaminosäure | | 0,03% |

## Revendications

1. Procédé de production d'une plante hybride d'Allium comprenant les étapes de: préparation de deux donneurs de protoplaste différents à partir de deux plantes d'Allium différentes, isolement des protoplastes à partir des donneurs, soumission des protoplastes à une fusion cellulaire pour fournir des cellules hybrides, où, après la fusion cellulaire, les cellules hybrides sont triées à partir des autres cellules puis cultivées séparément d'elles, caractérisé en ce que: après la fusion cellulaire et avant le tri des cellules, les cellules hybrides avec les autres cellules sont cultivées au moins dans une mesure telle que les parois cellulaires sont régénérées; et les deux plantes d'Allium sont l'oignon et l'ail, respectivement.

2. Procédé tel que défini dans la revendication 1, dans lequel, à titre d'étape préliminaire avant l'isolement des protoplastes, on plonge chacun des donneurs de protoplaste dans un milieu de culture.

3. Procédé tel que défini dans la revendication 2 dans lequel le milieu de culture pour l'étape préliminaire contient au moins de l'acide naphtalèneacétique (NAA) et de la benzyladénine (BA).

4. Procédé tel que défini dans une ou plusieurs des revendications 1-3, dans lequel chacun des donneurs de protoplaste est choisi dans le groupe constitué par une plante ayant connu une croissance normale, une jeune plante stérilisée obtenue en faisant incuber une partie de bulbe engendrant des feuilles, une plante dérivée d'un point de croissance de plante, une plante obtenue par redifférentiation d'un cal, un cal dérivé d'un point de croissance de plante, un cal dérivé d'un embryon, et un cal dérivé d'autres organes ou tissus d'une plante autre qu'un point de croissance et un embryon.

5. Procédé tel que défini dans une ou plusieurs des revendications 1-4, dans lequel on procède au tri des cellules hybrides en utilisant un microscope à fluorescence qui permet de distinguer les cellules hybrides en les colorant sous l'irradiation d'une lumière d'excitation bleue.

6. Procédé tel que défini dans une ou plusieurs des revendications 1 à 5, dans lequel, après le tri des cellules, on met les cellules hybrides dans un système de culture d'élevage, celui-ci comprenant un anneau à filtre disposé dans une boîte de Petri qui contient, autour de l'anneau à filtre, des protoplastes de culture pris dans une plante d'Allium ayant un potentiel élevé de division cellulaire, les cellules hybrides étant placées dans l'anneau à filtre pour la division cellulaire à élevage assisté.

7. Procédé tel que défini dans une ou plusieurs des revendications 1 à 6, dans lequel on conduit l'étape initiale de la culture de cellules hybrides en utilisant un milieu de culture 8p modifié [contenant de l'acide 2,4-dichlorophénoxyacétique (2,5 x 10⁻⁶ M)] ayant la composition représentée dans le Tableau I ci-dessous, le milieu de culture 8p modifié recevant progressivement tous les 4 à 5 jours un complément de milieu de culture BDS modifié [contenant de l'acide 2,4-dichlorophénoxyacétique (2,5 x 10⁻⁶ M)] ayant la composition représentée dans le Tableau II ci-dessous. 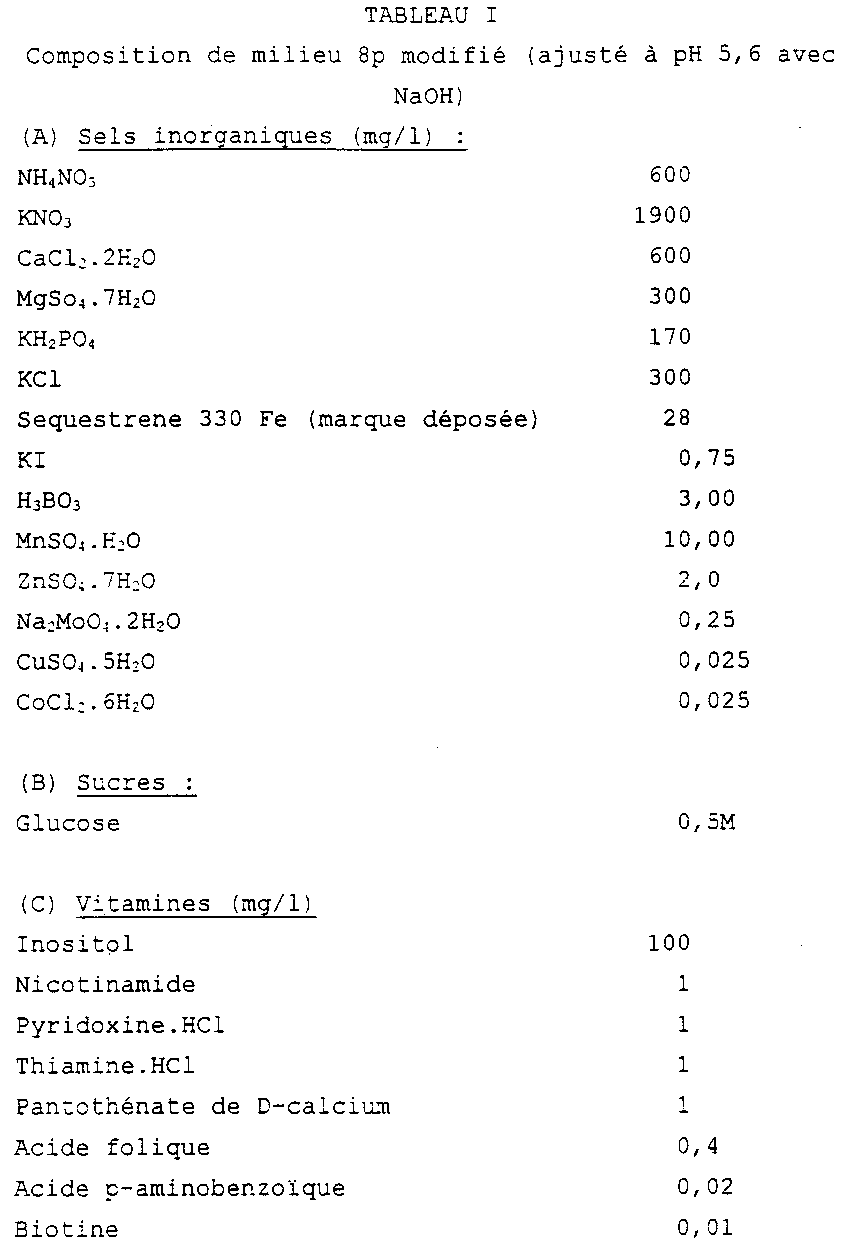 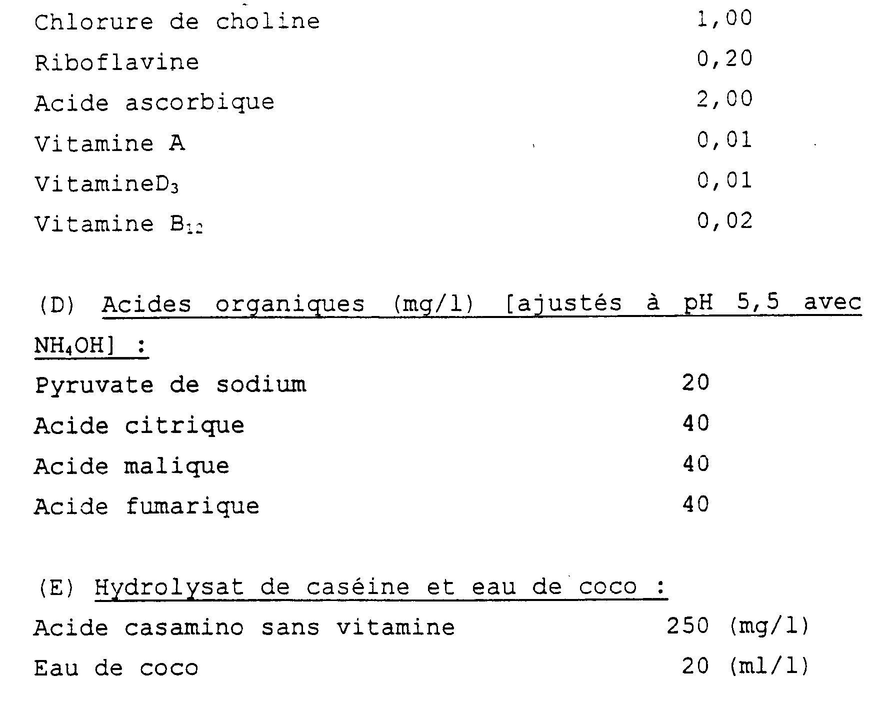
**Tableau II**
| Composition du milieu de culture BDS modifié (ajusté à pH 5,6 avec NaOH) | | |
|---|---|---|
| Composé | Poids | Concentration |
| CaCl₂.2H₂O | 150 mg | 1,02 mM |
| KNO₃ | 2,53 mg | 25,02 mM |
| NH₄NO₃ | 320,16 mg | 4,0 mM |
| NH₄H₂PO₄ | 230,06 mg | 2,0 mM |
| (NH₄)₂SO₄ | 134 mg | 1,01 mM |
| MgSO₄.7H₂O | 247 mg | 1,00 mM |
| MnSO₄.4H₂O | 13,2 mg | 0,045 mM |
| ZNSO₄.7H₂O | 2,0 mg | 6,95 µM |
| CuSO₄.5H₂O | 0,039 mg | 0,1 µM |
| KI | 0,75 mg | 4,52 µM |
| CoCl₂.6H₂O | 0,025 mg | 0,105 µM |
| H₃BO₃ | 3,0 mg | 0,049 µM |
| Na₂MoO₄.2H₂O | 0,25 mg | 1,03 µM |
| Na₂H₂PO₄.2H₂O | 172 mg | 1,04 µM |
| Fe₂SO₄.7H₂O | 27,85 mg | 100 µM |
| EDTA Na₂ | 37,25 mg | 100 µM |
| Acide nicotinique | 1,0 mg | 8,1 µM |
| Thiamine.HCl | 10 mg | 0,03 µM |
| Pyroxidine.HCl | 1,0 mg | 4,9 µM |
| Méso-inositol | 100 mg | 1,8 mM |
| Saccharose | 30 mg | 0,088 M |
| Acide casamino | | 0,03% |
